# EUROPÄISCHE PATENTANMELDUNG

(11) **EP 4 523 620 A1**
(43) Veröffentlichungstag der Anmeldung: **19.03.2025**
(21) Anmeldenummer: 23197566.5
(22) Anmeldetag: 15.09.2023
(51) Int. Cl.: A61B 5/107, G06T 7/62

(54) **BESTIMMUNG EINER LÄNGE EINES STENTS**

(71) Anmelder: Siemens Healthineers AG, 91301 Forchheim (DE)
(72) Erfinder: Pfister, Marcus, 91088 Bubenreuth (DE)
(74) Vertreter: Siemens Healthineers Patent Attorneys

(57) **Zusammenfassung**

Die Länge eines Stents für ein verzweigtes Gefäß soll möglichst ohne Kontrastmittel bestimmt werden. Dazu erfolgt ein Erfassen einer Projektionsaufnahme (37) des Gefäßes, in das ein Instrument (18, 19) eingeführt ist, ein Bestimmen des Instruments (18, 19) in der Projektionsaufnahme (37) und ein Schätzen einer 3D-Rekonstruktion des bestimmten Instruments (18, 19) aus der Projektionsaufnahme (37) zum Gewinnen eines virtuellen 3D-Instruments (28). Es erfolgt ein Festlegen eines Anfangspunkts (32) und eines Endpunkts (33) auf dem virtuellen 3D-Instrument (28) anhand einer Überlagerung des virtuellen 3D-Instruments (28) und der Projektionsaufnahme (37) und ein automatisches Bestimmen einer Distanz zwischen dem Anfangspunkt (32) und dem Endpunkt (33) entlang des virtuellen 3D-Instruments (28) als die Länge für den Stent.

## Beschreibung

Die vorliegende Erfindung betrifft ein Verfahren zum Bestimmen einer Länge für einen Stent für ein verzweigtes Gefäß. Darüber hinaus betrifft die vorliegende Erfindung eine entsprechende Anordnung zum Bestimmen dieser Länge. Ferner betrifft die vorliegende Erfindung auch ein korrespondierendes Computerprogramm.

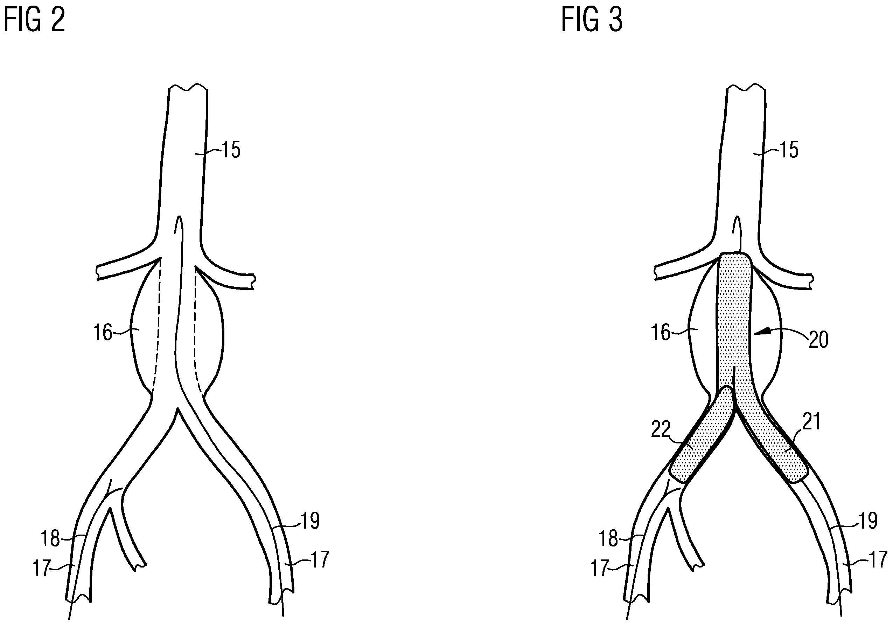

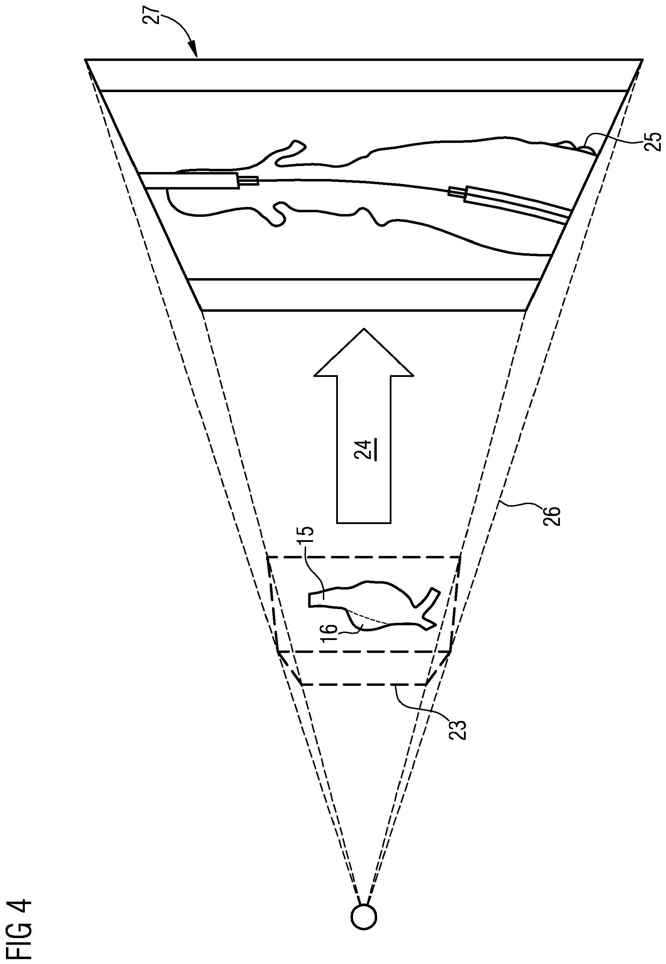

Ein abdominelles Aortenaneurysma (AAA) ist eine Gefäß-Aussackung an der abdominellen Aorta. Behandelt wird ein solches AAA durch Einsetzen verschiedener Stent Grafts (Gefäßplastiken). Über die beiden Leisten werden Führungsdrähte und Katheter in die Aorta eingebracht, über die zunächst ein Haupt(Aorten-)stent eingebracht wird, an den anschließend Stents für die Beinarterien (Iliaca) "angeflanscht" (vgl. FIG 3) werden. Bei komplexeren, sogenannten fenestrierten (mit Fenstern) oder gebranchten (verzweigten) Stents, kommen noch weitere Teil-Stents hinzu. Um zur Kontrolle während der komplexen Stent-Positionierung nicht zur ständigen Gefäßdarstellung Kontrastmittel injizieren zu müssen, kann zur Positionierungshilfe ein Referenzbild (anatomisch korrekt) überlagert werden, das die Gefäße (in dem Falle Aorta und abgehende Gefäße) darstellt (vgl. FIG 4).

Eine wesentliche Problematik besteht in dem Bestimmen der Länge der anzuflanschenden Iliacal-Stents. Diese Länge kann vor der Operation nur bedingt bestimmt werden, selbst bei einem vorsegmentierten und vermessenen Datensatz. Es ist zwar in der Regel die Länge der einzelnen Gefäße (z.B. als Länge der Centerlines) bekannt, allerdings nicht die spätere Position der Beinöffnungen des eingebrachten Aorten-Stents, die letztendlich erst während der Intervention (nach Absetzen des Stents) bekannt ist, da ja das Gefäß durch den eingebrachten Stent verformt wird. Zudem verkürzen sich die Iliacal-Arterien durch das Einbringen der steifen Instrumente (und Stents) erheblich. Beispielsweise wurde gemessen, dass sich die Länge der Iliaca durch die Verformung von präoperativ 66 ± 12 mm intraoperativ 60 ± 9 mm, verkürzt, im Mittel also um etwa 6 mm. Diese Verkürzung bleibt dann weitgehend bestehen.

Um beiden Effekten Rechnung zu tragen, wird als Standard nach eingebrachtem Aorten-Stent und Sondierung der Beinöffnung mit einem (steifen) Führungsdraht eine Angiographie mit einem speziellen Angio-Katheter mit Markierungen in lcm-Abstand gemacht. Anhand des "Abzählens" der Markierungen zwischen Iliacal-Bifurkation (Iliacal-Gabelung) und Bifurkation des eingebrachten Aorten-Stents wird dann die Länge des benötigten lliacal-Stents bestimmt.

In der Druckschrift DE 10 2013 219 737 B4 soll ein angiographisches Untersuchungsverfahren zur Darstellung eines Zielbereichs innerhalb eines Patienten mit einem Gefäßsystem derart ausgebildet werden, dass eine dreidimensionale Korrekturmöglichkeit aus nur einem Projektionsbild möglich ist, so dass sich der Verfahrensablauf und die Strahlenbelastung für den Patienten erheblich verbessern. Dazu wird ein Untersuchungsverfahren mit folgenden Schritten vorgeschlagen:
S1 Akquirierung eines Volumendatensatzes des Zielbereichs mit dem Untersuchungsobjekt,
S2 Registrierung des Volumendatensatzes zum C-Bogen,
S3 Extraktion von Informationen über einen angenommenen Verlauf des Untersuchungsobjekts im Volumendatensatz innerhalb des Zielbereichs
S4 Erzeugung wenigstens eines 2-D-Projektionsbildes eines im Zielbereich eingebrachten medizinischen Instruments, das eine Abweichung zwischen Überlagerung und tatsächlich projiziertem Instrument aufweist,
S5 2-D/3-D-Fusionierung des wenigstens einen 2-D-Projektionsbildes und des registrierten Volumendatensatzes zur Erzeugung eines 2-D-Überlagerungsbildes,
S6 Detektion des im Zielbereich eingebrachten Instruments im 2-D-Überlagerungsbild mit einer ersten Projektionsmatrix,
S7 Erstellung einer virtuellen 2-D-Projektion mittels einer virtuellen Projektionsmatrix,
S8 3-D-Rekonstruktion des Instruments, bei der dessen 3-D-Position aus den zwei Projektionen bestimmt wird und
S9 Überlagerung des 2-D-Projektionsbildes und der virtuellen 2-D-Projektion sowie Verzerrung zumindest eines Teils des 2-D-Projektionsbildes derart, dass der aktuelle und der angenommene Gefäßverlauf in Übereinstimmung gebracht werden. Mit diesem Ansatz ist die 3D-Rekonstruktion steifer Drähte in 3D aus einer Projektion durchführbar.

Druckschrift DE 10 2010 012 621 A1 beschreibt ein Schätzverfahren zum Ermitteln einer Kontur eines Gefäßes, das beispielsweise durch ein steifes Instrument (z.B. Führungsdraht) verformt wurde.

Die Aufgabe der vorliegenden Erfindung besteht somit darin, die Länge eines einzusetzenden Stents zu bestimmen und dabei die Strahlungsbelastung bzw. Kontrastmittelbelastung eines Objekts oder Patienten möglichst gering zu halten.

Erfindungsgemäß wird diese Aufgabe gelöst durch ein Verfahren und eine Anordnung entsprechend den unabhängigen Ansprüchen. Vorteilhafte Weiterbildungen der Erfindung ergeben sich aus den Unteransprüchen.

Erfindungsgemäß wird demnach ein Verfahren zum Bestimmen einer Länge für einen ersten Stent für ein verzweigtes Gefäß bereitgestellt. Beispielsweise soll in die abdominelle Aorta an der iliacalen Gabelung (Bifurkation) ein Stent Graft eingesetzt werden. Dabei ist beispielsweise in der Aorta bereits ein Stent eingesetzt (im vorliegenden Dokument als zweiter Stent bezeichnet) und für mindestens eine Beinarterie muss der genannte erste Stent eingefügt werden. Beide Stents werden miteinander verbunden. Damit der erste Stent z.B. nicht eine distale Abzweigung in der Beinarterie verdeckt, darf er nur eine bestimmte Länge besitzen. Hierfür wird die Länge für den ersten Stent bestimmt.

Hierzu erfolgt zunächst ein Erfassen einer Projektionsaufnahme des Gefäßes, in das ein (längliches) Instrument (z.B. Katheter, Führungsdraht) eingeführt ist. Bei der Projektionsaufnahme kann es sich beispielsweise um ein Durchleuchtungsbild handeln, das auf Röntgenbasis gewonnen wird. Die Projektionsaufnahme kann aber auch durch andere Durchleuchtungstechniken, insbesondere auch durch Sonographie und dergleichen gewonnen werden. Die Aufnahme kann aus einer bestimmten Richtung erfolgen.

Anschließend erfolgt ein Bestimmen (das ein automatisches Erkennen beinhalten oder sein kann) des Instruments in der Projektionsaufnahme. Beispielsweise handelt es sich bei dem Instrument um einen Katheter, der durch entsprechend geeignete Bildverarbeitung automatisch erkannt wird. Insbesondere kann seine Form und Ausdehnung registriert werden. Gegebenenfalls kann auch der Typ des Instruments erkannt werden (z. B. Führungsdraht). Das Instrument kann aber auch z.B. durch Setzen von Markern (ev. mit einer Detektionseinrichtung) bestimmt werden.

In einem weiteren Schritt erfolgt ein Durchführen bzw. Schätzen einer 3D-Rekonstruktion des bestimmten bzw. automatisch erkannten Instruments aus der Projektionsaufnahme zum Gewinnen eines virtuellen 3D-Instruments. Das bestimmte bzw. erkannte Instrument wird also in seiner Form dreidimensional rekonstruiert. Als Basis wird die (2D)-Projektionsaufnahme verwendet. Gegebenenfalls können für die 3D-Rekonstruktion des bestimmten Instruments auch eine oder mehrere zusätzliche Datensätze (z. B. Volumendatensatz des Gefäßes) herangezogen werden. Es genügt aber in der Regel eine einzige Projektionsaufnahme des Gefäßes mit dem eingeführten Instrument. Mit der 3D-Rekonstruktion liegt ein virtuelles 3D-Instrument in seiner dreidimensionalen Gestalt vor.

In einem weiteren Schritt werden ein Anfangspunkt und ein Endpunkt auf dem virtuellen 3D-Instrument anhand einer Überlagerung des 3D-Instruments und der Projektionsaufnahme festgelegt. Es wird also die reelle (2D)-Projektionsaufnahme mit dem virtuellen 3D-Instrument überlagert. Die Projektionsaufnahme zeigt in der Regel weitere Details des Gefäßes, wie etwa einen bereits eingesetzten zweiten Stent, eine Gabelung, eine Abzweigung und dergleichen. Diese Details beziehungsweise zusätzlichen Informationen können dazu verwendet werden, den Anfangspunkt und den Endpunkt auf dem virtuellen 3D-Instrument festzulegen. Beispielsweise soll als Anfangspunkt eine distale Öffnung des zweiten Stents und als Endpunkt eine Iliacal-Bifurkation (Satz, 5.50) für die Längenbestimmung dienen.

Schließlich erfolgt bei dem erfindungsgemäßen Verfahren ein automatisches Bestimmen einer Distanz zwischen dem Anfangspunkt und dem Endpunkt entlang des virtuellen 3D-Instruments als die Länge für den ersten Stent. Beispielsweise handelt es sich bei dem virtuellen 3D-Instrument um einen virtuellen Messkatheter, der in etwa die Steifigkeit eines Stents besitzt. Mit dem festgelegten Anfangs- und Endpunkt auf dem virtuellen 3D-Instrument lässt sich die Länge entlang des virtuellen Gebildes automatisch ermitteln. Diese Länge kann beispielsweise ein Arzt dazu verwenden, um die tatsächliche Länge eines einzusetzenden Stents zu ermitteln. Beispielsweise könnte der Arzt für den ersten Stent genau diejenige Länge verwenden, die automatisch bestimmt wird. Wünscht der Arzt aber beispielsweise eine Überlappung des ersten und zweiten Stents, so muss er zu der automatisch bestimmten Länge ein entsprechendes Überlappungsmaß hinzuaddieren, um zur letztlich gewünschten Länge des ersten Stents zu gelangen.

In vorteilhafter Weise ist es somit möglich, anstelle eines tatsächlichen Bilds mit einem Messkatheter eine Aufnahme mit einem virtuellen 3D-Instrument zu verwenden, um die Länge eines einzusetzenden Stents zu bestimmen. Auf diese Weise kann die Kontrastmittel- und Strahlungsbelastung eines Patienten reduziert werden.

In einem Ausführungsbeispiel ist vorgesehen, dass der Anfangspunkt an dem virtuellen 3D-Instrument an einer automatisch erkannten Öffnung eines zweiten Stents festgelegt wird. Wie oben erläutert, kann es sich bei dem zweiten Stent um einen Aortenstent handeln. Dieser Aortenstent kann beispielsweise einen tubusförmigen Hauptkörper aufweisen, an den sich distal kurze "Hosenbeine" (Limbs) für die Beinarterien anschließen. An die beiden "Hosenbeine" sollen anschließend Iliacal-Stents "angeflickt" werden. Beispielsweise besitzen die Iliacal-Stents eine Standardlänge von 60, 80 oder 100 mm. Durch die Längenmessung kann der geeignete Iliacal-Stent ausgewählt werden, gegebenenfalls unter Berücksichtigung einer Überlappung der beiden Stents von 1 bis 2 cm. Das automatische Erkennen der Öffnung (Hosenbeinöffnung) des zweiten Stents kann durch eine Bilderkennungssoftware erfolgen, die beispielsweise in der Projektion eine Ellipse der runden Öffnung erkennt.

In einem weiteren Ausführungsbeispiel ist vorgesehen, dass der Endpunkt an dem virtuellen 3D-Instrument anhand einer automatisch erkannten Abzweigung von dem Gefäß in der Projektionsaufnahme oder in einer über die Projektionsaufnahme überlagerten 3D-Kontur des Gefäßes festgelegt wird. Beispielsweise kann die oder eine andere Bildverarbeitungssoftware eben auch eine Gabelung des Gefäßes (z. B. Iliacal-Bifurkation) der Beinarterie in der 2D-Projektionsaufnahme erkennen und damit den Ort dieser Iliacal-Bifurkation als Endpunkt festlegen. Gegebenenfalls ist es günstiger, die Abzweigung in der (unter Umständen durch das Instrument verformten) 3D-Kontur des Gefäßes automatisch erkennen zu lassen.

Bei einer vorteilhaften Ausgestaltung des erfindungsgemäßen Verfahrens kann der Anfangspunkt oder der Endpunkt durch manuelles Markieren einer oder zweier entsprechender Stellen erfolgen. Beispielsweise wird auf dem Projektionsbild ein entsprechender Anfangspunkt und/oder Endpunkt manuell markiert und (gegebenenfalls automatisch) registriert. So kann beispielsweise durch Klicken auf eine entsprechende Stelle in einer Grafik eine Position des Anfangspunkts oder Endpunkts festgelegt werden. Unter Umständen lassen sich die markierten Stellen auch manuell verschieben. Unter Umständen können auch automatisch festgelegte Anfangs- und Endpunkte manuell korrigiert werden.

In einem anderen Ausführungsbeispiel kann vorgesehen sein, dass die Projektionsaufnahme die einzige für das Durchführen der 3D-Rekonstruktion des (manuell oder automatisch) bestimmten bzw. automatisch erkannten Instruments aus der Projektionsaufnahme zum Gewinnen des virtuellen 3D-Instruments ist. Dabei kann aus der (2D)-Projektionsaufnahme die dreidimensionale Gestalt des Instruments geschätzt werden. Für die 3D-Rekonstruktion kann man sich des Verfahrens bedienen, das eingangs in Zusammenhang mit der Druckschrift DE 10 2013 219 737 B4 erwähnt wurde. Insbesondere können für die Rekonstruktion somit die vorgeschlagenen Schritte S1 bis S9 durchgeführt werden.

In einem weiteren Ausführungsbeispiel ist vorgesehen, dass das Instrument ein Katheter, insbesondere ein Angio-Katheter, oder ein Führungsdraht ist. Das Instrument, das mit der Projektionsaufnahme aufgenommen wird, kann also beispielsweise ein Standard-Katheter sein. Insbesondere muss es sich nicht um einen goldenen Messkatheter handeln.

In einem weiteren Ausführungsbeispiel kann vorgesehen sein, dass das virtuelle 3D-Instrument einen virtuellen Messkatheter mit Messmarkierungen repräsentiert. Der virtuelle Messkatheter kann somit wie ein realer Messkatheter Messmarkierungen aufweisen, anhand denen der Arzt eine entsprechende Länge abschätzen kann. Darüber hinaus zeigen die Messmarkierungen dem Arzt auch etwaige dreidimensionale Verformungen in einer zweidimensionalen Projektionsaufnahme, nämlich wenn die Messmarkierungen näher beieinanderliegen als an anderen Stellen.

In einem speziellen Ausführungsbeispiel ist vorgesehen, dass das Gefäß eine Beinarterie beinhaltet, und der zweite Stent ein Aorten-Stent in einer abdominellen Aorta sowie die Abzweigung eine Iliacal-Bifurkation ist. Es wird also, wie in dem obigen Beispiel bereits erwähnt, der erste Stent in eine Beinarterie eingesetzt. Beginnen soll der erste Stent an dem Aorten-Stent in der abdominellen Aorta und enden sollte der erste Stent vor der Iliacal-Bifurkation. Mit einer derartigen Gefäßplastik kann ein abdominelles Aortenaneurysma behandelt werden.

In einem alternativen Ausführungsbeispiel ist vorgesehen, dass der erste Stent ein Intracranial-Stent ist. Das erfindungsgemäße Verfahren kann somit nicht nur für Iliacal-Stents verwendet werden, sondern auch für Stents innerhalb des Gehirns oder sonstiger Organe. Auch hier müssen Längen für Stents abgeschätzt werden, um deren Gesamtlänge oder Distanzen z.B. zu Fenestrierungen festzulegen.

Das erfindungsgemäße Verfahren kann also in einem Ausführungsbeispiel nicht nur für die Bestimmung der Gesamtlänge eines einzusetzenden Stents verwendet werden, sondern auch zur Bestimmung einer Distanz zu einer Stentöffnung oder zwischen zwei Stentöffnungen. Dazu ist für das erfindungsgemäße Verfahren vorgesehen, dass die Länge für den ersten Stent eine Distanz von einer Öffnung zu einem Fenster oder eine Distanz zwischen zwei Fenstern des ersten Stents ist. Somit können auch Stents für Nierenarterien insbesondere im Hinblick auf ihre Fenestrierungen leichter dimensioniert werden.

In einem weiteren Ausführungsbeispiel ist vorgesehen, dass eine Verformung des Gefäßes durch das Instrument modelliert und damit das Festlegen des Anfangspunkts und/oder Endpunkts durchgeführt wird. Dies bedeutet, dass das Gefäß durch das darin befindliche Instrument verformt wird, und das verformte Gefäß modelliert wird. Das Modellieren kann beispielsweise in einem Volumendatensatz erfolgen. Das modellierte, verformte Gefäß kann wiederum dazu verwendet werden, um beispielsweise in Kombination mit der 2D-Projektionsaufnahme den Anfangspunkt beziehungsweise Endpunkt für die Längenbestimmung festzulegen. Mit dem verformten Gefäßmodell lassen sich Anfangs- und Endpunkt zuverlässiger bestimmen.

In einem weiteren Ausführungsbeispiel besitzt der zweite Stent an der Öffnung eine Markierung, anhand der die Öffnung des zweiten Stents automatisch erkannt wird. Beispielsweise besitzt der Aorten-Stent an beiden "Hosenbeinöffnungen" Markierungen, die im Röntgenbild gut erkennbar sind. Damit kann beispielsweise der Anfangspunkt für die Länge des ersten Stents leichter festgelegt werden. Besondere Vorteile entstehen dann, wenn eine solche Markierung automatisch erkannt wird und die entsprechende Position auch automatisch festgelegt werden kann.

Gemäß einem weiteren Ausführungsbeispiel ist vorgesehen, dass der zweite Stent einen 3D-Marker besitzt, anhand dessen die Öffnung oder eine Bifurkation des zweiten Stents dreidimensional lokalisierbar ist. Beispielsweise ist der 3D-Marker an der Bifurkation des zweiten Stents angeordnet. Mit ihm sind in der Projektionsaufnahme die Öffnungen der "Hosenbeine" leichter zu orten, so dass damit gegebenenfalls auch ein Bestimmen bzw. automatisches Erkennen der Öffnung(en) ermöglicht wird.

Die oben genannte Aufgabe wird erfindungsgemäß auch gelöst durch eine Anordnung zum Bestimmen einer Länge für einen ersten Stent für ein verzweigtes Gefäß mit
- einer Erfassungseinrichtung, ausgebildet zum Erfassen einer Projektionsaufnahme des Gefäßes, in das ein (längliches) Instrument eingeführt ist,
- einer Detektionseinrichtung, ausgebildet zum Bestimmen des Instruments in der Projektionsaufnahme,
- einer Recheneinrichtung ausgebildet zum Schätzen (bzw. Durchführen) einer 3D-Rekonstruktion des bestimmten (z.B. automatisch erkannten) Instruments aus der Projektionsaufnahme zum Gewinnen eines virtuellen 3D-Instruments und
- einer Bestimmungseinrichtung, ausgebildet zum Festlegen eines Anfangspunkts und eines Endpunkts auf dem virtuellen 3D-Instrument anhand einer Überlagerung des virtuellen 3D-Instruments und der Projektionsaufnahme, wobei
- die Recheneinrichtung auch ausgebildet ist zum automatischen Bestimmen einer Distanz zwischen dem Anfangspunkt und dem Endpunkt entlang des virtuellen 3D-Instruments als die Länge für den ersten Stent.

Die Erfassungseinrichtung kann beispielsweise die Röntgentechnologie nutzen oder auf MRT oder Sonographie basieren. Die Detektionseinrichtung kann für das automatische Erkennen einen Bilderkennungsalgorithmus nutzen, der auf einem Computer beziehungsweise einer Recheneinrichtung einschließlich eines Prozessors implementiert ist. Darüber hinaus kann die Anordnung eine Recheneinrichtung für die 3D-Rekonstruktion nutzen. Die Detektionseinrichtung kann in die Recheneinrichtung integriert sein. Die Bestimmungseinrichtung kann ebenfalls in die Recheneinrichtung integriert sein, sofern eine automatische Bestimmung von Anfangspunkt und Endpunkt möglich ist. Alternativ kann die Bestimmungseinrichtung auch eine Benutzerschnittstelle umfassen, mit der ein Benutzer Anfangs- und Endpunkte setzen beziehungsweise markieren kann.

Des Weiteren wird entsprechend der vorliegenden Erfindung ein Computerprogramm bereitgestellt, das Befehle aufweist, welche, wenn sie in einem Prozessor der oben genannten Anordnung ausgeführt werden, diese veranlassen, das oben geschilderte Verfahren auszuführen.

Für Anwendungsfälle oder Anwendungssituationen, die sich bei dem Verfahren ergeben können und die hier nicht explizit beschrieben sind, kann vorgesehen sein, dass gemäß dem Verfahren eine Fehlermeldung und/oder eine Aufforderung zur Eingabe einer Nutzerrückmeldung ausgegeben und/oder eine Standardeinstellung und/oder ein vorbestimmter Initialzustand eingestellt wird.

Unabhängig vom grammatikalischen Geschlecht eines bestimmten Begriffes sind Personen mit männlicher, weiblicher oder anderer Geschlechteridentität mit umfasst.

Die vorliegende Erfindung wird nun anhand der beigefügten Zeichnungen näher erläutert, in denen zeigen:
- FIG 1: ein bekanntes C-Bogen-Angiographiesystem mit einem Industrieroboter als Tragvorrichtung,
- FIG 2: eine abdominale Aorta mit einem Aorten-Aneurysma,
- FIG 3: die Aorta gemäß FIG 2 mit eingeführtem Stent-Graft,
- FIG 4: eine Darstellung zur Erläuterung des Prinzips einer 2D-3D-Überlagerung,
- FIG 5: die automatische Erkennung eines eingebrachten Drahts und dessen 3D-Rekonstruktion; und
- FIG 6: das Festlegen von Anfangs- und Endpunkt für die automatische Längenbestimmung.

Die nachfolgend näher geschilderten Ausführungsbeispiele stellen bevorzugte Ausführungsformen der vorliegenden Erfindung dar.

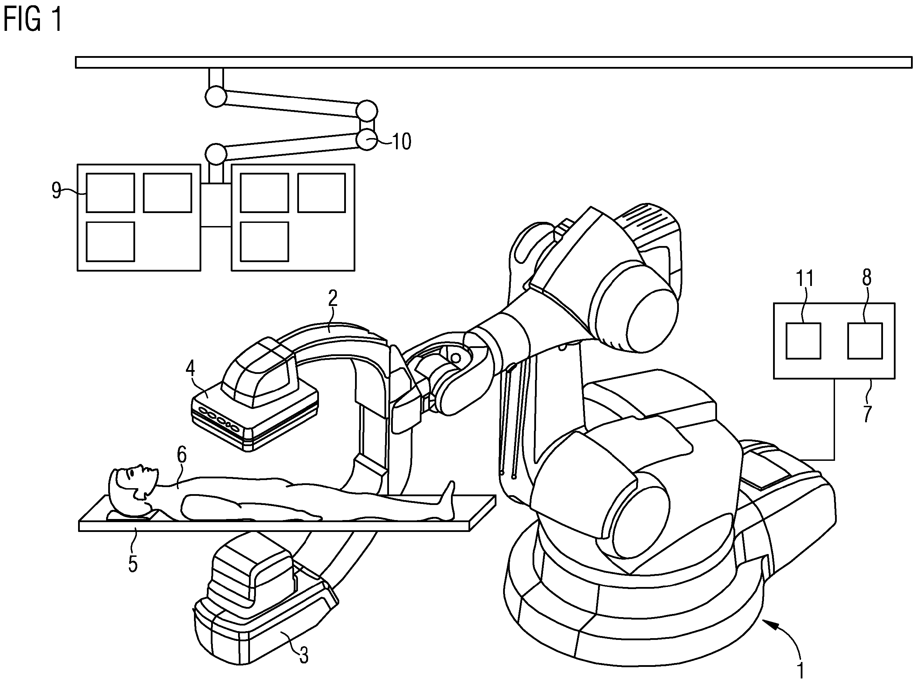

Die FIG 1 zeigt ein als Beispiel dargestelltes monoplanes Röntgensystem mit einem von einem Ständer 1 in Form eines sechsachsigen Industrie- oder Knickarmroboters gehaltenen C-Bogen 2, an dessen Enden eine Röntgenstrahlungsquelle, beispielsweise ein Röntgenstrahler 3 mit Röntgenröhre und Kollimator, und ein Röntgenbilddetektor 4 als Bildaufnahmeeinheit angebracht sind. Die Realisierung der Röntgendiagnostikeinrichtung ist nicht auf den Industrieroboter angewiesen. Es können auch übliche C-Bogen-Geräte Verwendung finden.

Im Strahlengang des Röntgenstrahlers 3 befindet sich auf einer Tischplatte 5 eines Patientenlagerungstisches ein zu untersuchender Patient 6 oder ein technischer Gegenstand als Untersuchungsobjekt. An der Röntgendiagnostikeinrichtung ist eine Systemsteuerungseinheit 7 mit einer Recheneinrichtung 8 zum Gewinnen eines virtuellen 3D-Instruments angeschlossen, das die Bildsignale des Röntgenbilddetektors 4 empfängt und verarbeitet (Bedienelemente sind beispielsweise nicht dargestellt). Die Röntgenbilder können dann auf Displays einer Monitorampel 9 betrachtet werden. Die Monitorampel 9 kann mittels eines deckenmontierten, längs verfahrbaren, schwenk-, dreh- und höhenverstellbaren Trägersystems 10 mit Ausleger und absenkbarem Tragarm gehalten werden. In der Systemsteuerungseinheit 7 ist weiterhin eine Bestimmungseinrichtung 11 zum Festlegen eines Anfangspunkts und eines Endpunkts auf dem virtuellen 3D-Instrument vorgesehen.

In FIG 2 ist eine abdominale Aorta 15 dargestellt, die ein abdominales Aortenaneurysma (AAA) 16 aufweist. Ein abdominales Aortenaneurysma (AAA) 16 ist eine Gefäßaussackung an der abdominalen Aorta 15. Die Aorta 15 verzweigt sich in die Beinarterien 17 Arteria iliaca communis.

Behandelt wird das Aortenaneurysma 16 durch Einsetzen eines Stent-Grafts, also einer Gefäßplastik, wie dies in FIG 3 dargestellt ist. Dazu werden über die beiden Leisten durch die Beinarterien 17 Führungsdrähte 18 und Katheter 19 in die Aorta 15 eingebracht, mittels denen die Stent-Grafts 20 eingesetzt werden.

Bei komplexen Stent-Grafts 20, die die Beinarterien 17 mit umfassen, muss der endgültige Stent manchmal aus "Teilstents" zusammengesetzt werden, wobei zum Beispiel an einem Aortenstent 21 als Hauptstent (zweiter Stent), der durch das AAA in eine der Beinarterien 17 ragt, durch ein sogenanntes Fenster (Öffnung) ein Iliacalstent 22 als Teilstent (erster Stent) für die andere Beinarterie "angeflanscht" wird.

Um dem Arzt auch heute schon zusätzliche Informationen zur Unterstützung beim Setzen von AAA-Stents zu geben, wird einem mittels eines C-Bogen-Systems 2 bis 4 erstellten aktuellen Durchleuchtungsbild ein vorher aufgenommenes Referenzbild anatomisch korrekt überlagert. Dieses Referenzbild kann ein 3D-Datensatz bzw. Volumendatensatz der Aorta 15 mit dem abdominalen Aortenaneurysma 16 gemäß FIG 3 sein, beispielsweise eine vorsegmentierte präoperative Computertomographie oder Rotationsangiographie mittels C-Bogen-Angiographiesystems.

Die FIG 4 veranschaulicht nun die Überlagerung eines aktuellen Durchleuchtungsbildes mit dem prä-interventionell erzeugten Volumendatensatz 23, der beispielsweise als 3D-Gittermodell vorliegen kann, wie dies in dem Würfel exemplarisch dargestellt ist. Das 3D-Gittermodell wird durch 3D-Projektion 24 in das Durchleuchtungsbild als 2D-Segmentierung 25 abgebildet, wie dies durch die gepunkteten Linien 26 symbolisiert wird, und man erhält ein 2D/ 3D-Überlagerungsbild 27 als Referenzbild.

Entsprechend einem Ausführungsbeispiel der vorliegenden Erfindung wird beispielsweise eine Längenschätzung durch eine Kombination von Informationen aus einem präoperativen (vorsegmentierten) CT und einem intraoperativen Durchleuchtungsbild möglich. Dazu wird beispielsweise ein durch CT gewonnener und zum Angiographiesystem beziehungsweise C-Arm registrierter 3D-Datensatz der Aorta und der Iliacal-Gefäße gewonnen. Außerdem sollten Informationen über Gefäßabgänge in diesem Datensatz, insbesondere der Iliaca-interna-Abgänge vorhanden sein. Ferner sollte in die Aorta der Haupt- oder Aortenstent (zweiter Stent) eingebracht sein. Der 3D-Datensatz kann somit z. B. eine präoperative (zum C-Arm registrierte) CT- oder MR-Angiographie sein oder auch eine intraoperative 3D-Bildgebung.

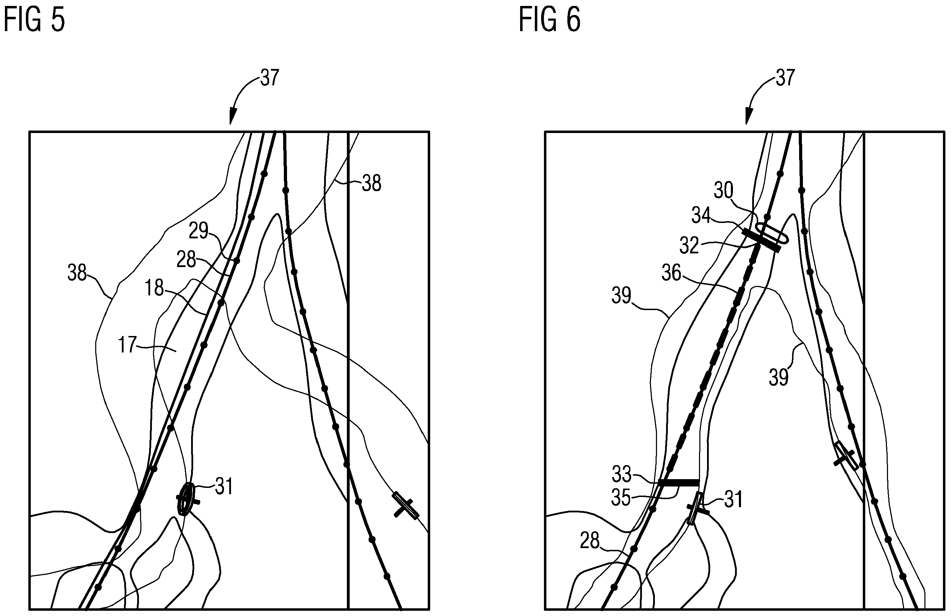

Der nachfolgend beschriebene Workflow könnte erfindungsgemäß zur Bestimmung der Stentlänge des ersten Stents (Teilstent) verwendet werden. Die Bestimmung der Stentlänge kann insbesondere kontrastmittelfrei und intraoperativ erfolgen. Der Workflow beinhaltet folgende Schritte:
1. Aufnahme eines Durchleuchtungsbilds (Projektionsaufnahme) aus der vom Arzt bevorzugten Projektionsrichtung (Hintergrund von FIG 5) .
2. Automatische Erkennung des in die Beinarterie 17 eingebrachten Drahtes 18 in dem Durchleuchtungsbild. Dies kann beispielsweise durch entsprechende Bilderkennungssoftware erfolgen.
3. 3D-Rekonstruktion des (verformten) Instruments. Bei dieser 3D-Rekonstruktion ergibt sich ein virtuelles 3D-Instrument 28 aus der (2D)-Projektion beziehungsweise Projektionsaufnahme. Diese 3D-Rekonstruktion kann gemäß der eingangs erwähnten Druckschrift DE 10 2013 219 737 B4 erfolgen. Insbesondere können hierzu die Schritte S1 bis S9 verwendet werden. Beispielsweise entspricht das virtuelle 3D-Instrument einem durch das Gefäß verformten Draht. Gegebenenfalls wird das virtuelle 3D-Instrument (hier der virtuelle 3D-Draht) gemäß FIG 5 mit Längenmarkierungen auf einem Monitor dargestellt. Eine Verformung des virtuellen Drahts 28 in z-Richtung (senkrecht zur Bildebene) könnte man daran erkennen, dass die Längenmarkierungen 29 dann näher beieinanderliegen.
4. Automatische Erkennung des eingebrachten (zweiten) Stents beziehungsweise dessen distaler (d. h. vom Herzen weiter entfernten) Öffnung 30 (vgl. FIG 6). Für diese automatische Erkennung kann wiederum eine Bilderkennungssoftware verwendet werden.
5. Automatische Distanzbestimmung zwischen der automatisch erkannten Öffnung 30 des (zweiten) Stents und einer gegebenenfalls ebenso automatisch erkannten Bifurkation 31 des Gefäßes entlang des (gekrümmten) virtuellen 3D-Instruments. Als Anfangspunkt 32 wird beispielsweise ein Schnittpunkt einer geschätzten Querschnittslinie 34 durch das Gefäß an der Öffnung 30 beziehungsweise in unmittelbarer Nähe von ihr mit dem virtuellen 3D-Instrument beziehungsweise -Draht verwendet. Als Endpunkt 33 wird beispielsweise ein Schnittpunkt zwischen einer Querschnittslinie 35 und dem virtuellen Draht 28 an der Bifurkation beziehungsweise in der unmittelbaren Nähe davon verwendet. Zwischen Anfangspunkt 32 und Endpunkt 33 ergibt sich der in FIG 6 gestrichelt eingezeichnete Abschnitt 36 des virtuellen 3D-Instruments 28, der die zu bestimmende Länge repräsentiert.
6. Optional kann das Durchleuchtungsbild beziehungsweise die Projektionsaufnahme 37, 37' mit einer Kontur 38 des Gefäßes überlagert werden, damit sich der behandelnde Arzt besser orientieren kann. In FIG 5 ist die Projektionsaufnahme 37 mit der unverformten Kontur 38 des Gefäßes überlagert, in das der erste Stent einzubringen ist. Es ist zu erkennen, dass der Führungsdraht 18 teilweise außerhalb der Kontur 38 des Gefäßes verläuft. Dies liegt daran, dass bei der Kontur 38 die Gefäßverformung nicht berücksichtigt ist, die sich durch den Führungsdraht 18 ergibt. Daher kann zur besseren Orientierung des Arztes die Verformung des Gefäßes geschätzt werden. Wird nun gemäß FIG 6 die Kontur 39 des verformten Gefäßes der Projektionsaufnahme 37 überlagert, so ist zu erkennen, dass der Führungsdraht 18 beziehungsweise das virtuelle 3D-Instrument 28 innerhalb der Kontur 39 des verformten Gefäßes verläuft. Die Schätzung der Gefäßverformung kann gemäß der eingangs erwähnten Druckschrift DE 10 2010 012 621 A1 erfolgen. Der Arzt kann sich somit während der Intervention besser orientieren.

In einem alternativen Ausführungsbeispiel können der Anfangspunkt 32 und der Endpunkt 33 auch durch manuelles Markieren beispielsweise auf der Projektionsaufnahme 37 gesetzt werden. Das manuelle Markieren kann durch "Klicken" auf die entsprechende Stelle im Durchleuchtungsbild beziehungsweise der Projektionsaufnahme 37 erfolgen.

In einer weiteren Ausführungsform kann an dem Aortenstent (zweiter Stent) an der Bifurkation auch eine spezielle Markierung angebracht sein, die eine automatische Erkennung im 2D-Bild (Projektionsaufnahme 37) vereinfacht. Insbesondere kann diese Markierung im Bereich der Öffnung 30 (vgl. FIG 6) angebracht sein.

In einem weiteren Ausführungsbeispiel kann an dem Aortenstent (zweiter Stent) (z. B. an der Bifurkation) auch ein 3D-Marker angebracht sein, der eine Lokalisierung der Bifurkation in 3D erlaubt. Damit kann eine Überlagerung einer 2D-Projektionsaufnahme und einer 3D-Rekonstruktion des Gefäßes noch präziser erfolgen.

Gemäß einer weiteren Ausführungsform kann anstatt der distalen Öffnung an der Stent-Bifurkation ein beliebiger anderer Punkt markiert oder automatisch erkannt werden, der zur Berechnung der gesuchten Länge führen kann. Der andere Punkt sollte aber auch auf dem virtuellen 3D-Instrument (virtueller Draht) liegen, damit die Längenbestimmung sinnvoll entlang des 3D-Instruments erfolgen kann.

Das beschriebene Verfahren ist nicht auf die Berechnung der Länge von Iliacalstents beschränkt, sondern kann entsprechend auch auf die Berechnung der Abstände von Fenestrierungen (z. B. Stents für Nierenarterien) oder die Berechnung der Länge von Intracranialstents verwendet werden.

Entsprechend den oben vorgestellten Ausführungsbeispielen ist es möglich, intraoperativ ohne Kontrastmittel die Länge von Abschnitten von Gefäßen zu bestimmen, um letztlich entsprechende Stentlängen zu ermitteln.

## Patentansprüche

1. Verfahren zum Bestimmen einer Länge für einen ersten Stent (22) für ein verzweigtes Gefäß (15, 17) durch
- Erfassen einer Projektionsaufnahme (37) des Gefäßes (15, 17), in das ein Instrument (18, 19) eingeführt ist,
- Bestimmen des Instruments (18, 19) in der Projektionsaufnahme,
- Schätzen einer 3D-Rekonstruktion des bestimmten Instruments (18, 19) aus der Projektionsaufnahme (37) zum Gewinnen eines virtuellen 3D-Instruments (28),
- Festlegen eines Anfangspunkts (32) und eines Endpunkts (33) auf dem virtuellen 3D-Instrument (28) anhand einer Überlagerung des virtuellen 3D-Instruments (28) und der Projektionsaufnahme (37) und
- automatisches Bestimmen einer Distanz zwischen dem Anfangspunkt (32) und dem Endpunkt (33) entlang des virtuellen 3D-Instruments (28) als die Länge für den ersten Stent (22).

2. Verfahren nach Anspruch 1, wobei der Anfangspunkt (32) an dem virtuellen 3D-Instrument (28) anhand einer automatisch erkannten Öffnung (30) eines zweiten Stents (21) festgelegt wird.

3. Verfahren nach Anspruch 1 oder 2, wobei der Endpunkt (33) an dem virtuellen 3D-Instrument (28) anhand einer automatisch erkannten Abzweigung (31) von dem Gefäß (15, 17) in der Projektionsaufnahme (37) oder in einer über die Projektionsaufnahme (37) überlagerten 3D-Kontur (38) festgelegt wird.

4. Verfahren nach einem der vorhergehenden Ansprüche, wobei das Festlegen des Anfangspunkts (32) oder des Endpunkts (33) durch manuelles Markieren von einer oder zwei entsprechenden Stellen erfolgt.

5. Verfahren nach einem der vorhergehenden Ansprüche, wobei die Projektionsaufnahme (37) die einzige für das Durchführen der 3D-Rekonstruktion des bestimmten Instruments (18, 19) aus der Projektionsaufnahme (37) zum Gewinnen des virtuellen 3D-Instruments (28) ist.

6. Verfahren nach einem der vorhergehenden Ansprüche, wobei das Instrument (18, 19) ein Katheter (19), insbesondere ein Angio-Katheter, oder ein Führungsdraht (18) ist.

7. Verfahren nach einem der vorhergehenden Ansprüche, wobei das virtuelle 3D-Instrument (28) einen virtuellen Messkatheter mit Messmarkierungen repräsentiert.

8. Verfahren nach Anspruch 2 und 3, wobei das Gefäß (15, 17) eine Beinarterie beinhaltet, und der zweite Stent (21) ein Aorten-Stent in einer abdominellen Aorta sowie die Abzweigung eine Iliacal-Bifurkation ist.

9. Verfahren nach einem der vorhergehenden Ansprüche, wobei der erste Stent (22) ein Iliacalstent oder ein Intracranialstent ist.

10. Verfahren nach einem der vorhergehenden Ansprüche, wobei die Länge für den ersten Stent (22) eine Distanz von einer Öffnung (30) zu einem Fenster oder eine Distanz zwischen zwei Fenstern des ersten Stents (22) ist.

11. Verfahren nach einem der vorhergehenden Ansprüche, wobei eine Verformung des Gefäßes (15, 17) durch das Instrument (18, 19) modelliert und damit das Festlegen des Anfangspunkts (32) und/oder Endpunkts (33) durchgeführt wird.

12. Verfahren nach Anspruch 2, wobei der zweite Stent (21) an der Öffnung (30) eine Markierung besitzt, anhand der die Öffnung (30) des zweiten Stents (21) automatisch erkannt wird.

13. Verfahren nach Anspruch 2, wobei der zweite Stent (21) einen 3D-Marker besitzt, anhand dessen die Öffnung (30) oder eine Bifurkation des zweiten Stents (21) dreidimensional lokalisierbar ist.

14. Anordnung zum Bestimmen einer Länge für einen ersten Stent (22) für ein verzweigtes Gefäß (15, 17) mit
- einer Erfassungseinrichtung (1 bis 4), ausgebildet zum Erfassen einer Projektionsaufnahme (37) des Gefäßes (15, 17), in das ein Instrument (18, 19) eingeführt ist,
- einer Detektionseinrichtung, ausgebildet zum Bestimmen des Instruments (18, 19) in der Projektionsaufnahme (37),
- einer Recheneinrichtung (8) ausgebildet zum Schätzen einer 3D-Rekonstruktion des bestimmten Instruments (18, 19) aus der Projektionsaufnahme (37) zum Gewinnen eines virtuellen 3D-Instruments (28) und
- einer Bestimmungseinrichtung (11), ausgebildet zum Festlegen eines Anfangspunkts (32) und eines Endpunkts (33) auf dem virtuellen 3D-Instrument (28) anhand einer Überlagerung des virtuellen 3D-Instruments (28) und der Projektionsaufnahme (37), wobei
- die Recheneinrichtung (8) auch ausgebildet ist zum automatischen Bestimmen einer Distanz zwischen dem Anfangspunkt (32) und dem Endpunkt (33) entlang des virtuellen 3D-Instruments (28) als die Länge für den ersten Stent (22).

15. Computerprogramm, das Befehle aufweist, welche, wenn sie in einem Prozessor der Anordnung nach Anspruch 14 ausgeführt werden, diese veranlassen, ein Verfahren nach einem der Ansprüche 1 bis 13 auszuführen.
